# EUROPEAN PATENT SPECIFICATION

(11) **EP 2 001 398 B1**
(45) Date of publication and mention of the grant of the patent: **26.05.2010**
(21) Application number: 07736672.2
(22) Date of filing: 07.03.2007
(51) Int. Cl.: A61F 2/00, A61B 17/04, A61B 17/06

(54) **PROSTHESIS FOR THE TREATMENT OF URINARY INCONTINENCE**
PROTHESE ZUR BEHANDLUNG DER HARNINKONTINENZ
PROTHÈSE POUR LE TRAITEMENT DE L'INCONTINENCE URINAIRE

(43) Date of publication of application: 17.12.2008
(73) Proprietor: Assut Europe S.P.A., 67062 Magliano dei Marsi AQ (IT)
(72) Inventor: D'Afiero, Alessandro, I-80026 Casoria NA (IT); LONGO, Maurizio, I-00157 Roma (IT)
(74) Representative: Papa, Elisabetta
(86) International application number: PCT/IT2007/000167
(87) International publication number: WO 2008/107919

(56) References cited:
- EP-A1- 1 800 619
- WO-A-2005/112842
- WO-A-2006/108145
- WO-A1-02/078552
- WO-A2-2007/149555
- US-A- 6 042 534
- US-A1- 2002 058 959
- US-A1- 2005 004 576
- US-A1- 2005 267 531

## Description

The present invention refers to a prosthesis to be used for surgical therapy of female urinary incontinence and urogenital prolapse.

### Background of the Invention

By urinary incontinence it is meant, in general, involuntary loss of urine through the urethra, which evidently causes hygienic, economic and social problems.

The subject-matter of the present invention concerns the treatment of *stress* urinary incontinence (SUI), consisting in the loss of urine consequent to increases of the abdominal pressure caused by coughs, sneezes, lifting of weights, etc.; a condition this occurring when bladder pressure exceeds urethra pressure, in the absence of normal compensation mechanisms.

Stress urinary incontinence may be related to alterations of pelvic floor muscles, or to an intrinsic urinary deficit. Factors that can cause said pathology are advanced age, delivery, pregnancy, menopause, smoking and obesity.

Three degrees of SUI may be distinguished, and precisely:
- first degree: losses of urine when coughing, laughing or sneezing;
- second degree: losses of urine when lifting weights or climbing stairs;
- third degree: losses of urine when standing, but not when lying down.

Urogenital prolapse is a pathology characterized by the inferior displacement of the vagina and uterus, accompanied by dislocation of the adjacent pelvic viscera, due to a sagging of their supporting means. Urogenital prolapse can be caused by delivery-provoked relaxation, menopause, but also by a chronic constipation.

Prolapse may concern all organs in the pelvic cavity: the uterus, the vagina, the bladder and the rectum. These organs are held inside the pelvis thanks to the support by the pelvic floor muscles and to a suspension system formed by connective tissue. As years go by, muscles become less tonic and the connective layer grows thin, causing the sagging. It may also happen that the expulsive phase of delivery weakens these structures and, always with age advancing, relaxation gets to cause the prolapse. Moreover, the continual insult to muscles caused by a chronic constipation ends up by decreasing muscle tonicity.

Urogenital prolapse entails a general worsening of the quality of life, causing various disturbances among which back pain, feeling of weight in the lower abdomen and local circulatory disturbances. The prevalence of urogenital prolapse is relevant among 15% to 35% of women, mostly in their fifties and sixties. The main clinical manifestation is an uncomfortable and bothersome feeling of protrusion or weight, localized at the level of the pelvis or of the perineum, and especially at the level of the vagina. These symptoms are worsened by the standing position, above all when prolonged.

SUI phenomenon may be caused by urogenital prolapse, it being generated by a descent of the anterior wall of the vagina, the urethra and the bladder neck. Hence, often to urogenital prolapse also SUI is associated.

In literature, several surgical methodologies are known for the treatment of urogenital prolapse and stress urinary incontinence. Aim of the surgical operation is to improve woman's quality of life, therefore it is envisaged only in case said quality is limited just by said pathologies.

A surgical approach used for the correction of the urogenital prolapse is colpohysterectomy (removal of the uterus through the vagina), associated to anterior colporrhaphy. Anterior colporrhaphy, commonly known as "vagina plastic surgery" envisages the removal of redundant vaginal wall.

In case of SUI associated to urogenital prolapse, beside the anterior colporrhaphy, SUI correction is performed by urethropexy. Such a technique entails the attachment of the urethra, at the ventral abdominal wall thereof, to the cranial end of the pubis, causing relocation of the bladder neck to an intrabdominal position and localized increase of urethral strength. However, it does not offer a high percentage of success, as at + 6 months - 1 year after operation recurrence phenomena have been observed in about the 30% of subjects subjected to said surgical treatment. Defects of discussed techniques are to be sought in the fact that in aged persons (like those subjected to this kind of operations) the collagen tissue formed following the operation is of a weak type, itself constituting a predisposition for a subsequent prolapse of the surgically treated structures.

More recently, in urogynaecologic practice an innovative surgical technique has been introduced referred to as TVT (Tension-free Vaginal Tape). It is a technique utilizing a prosthesis formed by a mesh (commonly denominated *"sling")* of non-reabsorbable or partially reabsorbable polypropylene applied, by "Tension Free" technique in a mini-invasive manner, on a position beneath the urethra, for supporting the same. The Tension Free technique is such that the inserted prosthesis does not exert direct traction on surrounding tissues, exploiting instead fibrosis phenomena developing shortly after implantation and tending to incorporate and hold *in situ* the prosthesis itself. TVT may be used to solve both the SUI problem and the urogenital prolapse problem.

International Application WO 2004/098461 describes a prosthesis to be used in the surgical therapy of urogenital prolapse and urinary incontinence according to said TVT technique Such a prosthesis is constituted by a central body, made of non-reabsorbable or partially reabsorbable polypropylene mesh, comprising a first portion to be placed beneath the bladder neck and middle urethra and a second portion to be applied in correspondence of the body and base of the bladder. From said central body, two pairs of arms (always made of polypropylene mesh) extend to create, with "Tension Free" technique, an elastic support for supporting (preventing their future descent) the pelvic structures subjected to prolapse and incontinence. For prosthesis insertion, the surgical operation envisages the execution of incisions at the pelvic level for the insertion/extraction of curved needles for maneuvering, attached just to said arms.

A further commercial product *("Gynecare TVT Secur* ®*"),* made to treat exclusively SUI, is constituted by a prosthesis made of a non-reabsorbable or partially reabsorbable polypropylene mesh, yet devoid of arms, unlike the prosthesis described in International Pat. Appl. WO 2004/098461. In fact, in this case the supporting of the pelvic structures is carried out by the body of the sling itself, however having a length of about 8 cm, and therefore remarkably reduced with respect to similar prostheses present in literature. The operation for the insertion of such a product requires no execution of incisions, it being directly inserted via the vagina.

US 6, 042,534 discloses a prefabricated urethral suspension slings made of biocompatible material and having lateral suture receiving sites thicker than the central portion.

WO 2006/108145 discloses various embodiments of surgical implants for the treatment of pelvic floor disorders or urinary incontinence. In one embodiment, the surgical implant has, at each opposite longitudinal end thereof, an elastic ring which couples the implant body to a respective soft tissue anchor. The elastic rings are connected to the implant body by dedicated through-apertures.

EP 1 800 619 discloses a mesh for the surgical treatment of urinary incontinence. The mesh body is associated with short threads passed through the gaps of the mesh itself. Such threads can be passed outside cutaneous suture lines and pulled for tightening/loosing the mesh after the intervention.

WO 02/078552 discloses an artificial ligament or tie between internal parts in a human body, also in case of vault or vaginal prolapse. The artificial ligament is made of a strip within which is looped a drawstring which can be pulled to shorten the strip.

### Drawbacks of the Prior Art

Referring to the prosthesis described in International Pat. Appl. WO 2004/09846, and generally to all prosthesis types similar thereto known in literature, the main drawback of the known art resides in the fact that the material left inside the body is clearly more than what is actually needed. In fact, the extension of the polypropylene mesh used (prosthesis body + arms) may be cause of complications that can be found during the surgical operation itself or subsequently, like, e.g., infections, erosions and rejection.

On the other hand, the *Gynecare*® product, by being constituted only of a sling, though of reduced dimensions, entails the advantage of drastically reducing the material inserted into the body, along with the fact of being inserted with no need to execute surgical incisions on the patient. Its remarkable drawback lies however in its limited supporting capability, associated just to the limited dimensions of the body and to its reduced "maneuvering" option - i.e., of adjusting the prosthesis position and tensioning level - during the insertion, related to the absence of arms. In fact, referring to the three degrees of seriousness of urinary incontinence, said prosthesis is capable of curing said pathology until the second level of seriousness only.

### Summary of the Invention

Hence, the technical problem set and solved by the present invention is to overcome the drawbacks hereto-mentioned with reference to the known art.

Such a problem is solved by a prosthesis according to claim 1.

Preferred features of the invention are provided in the dependent claims hereinafter.

The prosthesis subject-matter of the present invention proposes the union of a main prosthetic body - typically made of a synthetic or biologic non-reabsorbable or partially reabsorbable mesh - with special and preferably self-locking thread-shaped members for the *in situ* positioning, with the desired tension level, of the prosthesis. Such thread-shaped members are adopted in lieu of the arms of considerable extension provided in some known prostheses, and are them also partially or totally absorbable or not absorbable.

The adoption of such thread-shaped members allows to remarkably reduce the material used, which is subsequently left inside the patient. Thus, it is prevented any short- and long-term complication hereto-mentioned with reference to the known art, though attaining a surgical aid suitable for the treatment of stress urinary incontinences of any level, as well as of urogenital prolapse.

Moreover, the thread-shaped structure of the members intended for positioning and, at least initially, *in situ* holding of the prosthesis, allows a greater simplicity of surgical operation, a reduction of operating times and therefore of anesthesia times and amounts and a limited need of dissection with respect to what to date is envisaged in the art in order to implant the known prostheses.

Therefore, the prosthesis subject-matter of the present invention is truly mini-invasive.

### Brief Description of the Figures

Other advantages, features and the operation modes of the present invention will be made apparent from the following detailed description of some embodiments thereof, given by way of example and not for limitative purposes. Reference will be made to the figures of the annexed drawings, wherein:
- figure 1 shows a top perspective view of a prosthesis according to a first preferred embodiment of the present invention;
- figures 2 and 3 show each a perspective enlarged view of the prosthesis of figure 1, depicting an embodiment detail thereof;
- figure 4 schematically shows the last phase of the surgical technique for implantation of the prosthesis of figure 1;
- figure 5 shows a top perspective view of a prosthesis according to a second preferred embodiment of the present invention; and
- figure 6 schematically shows the last phase of the surgical technique for the application of the prosthesis of figure 5.

### Detailed Description of the Invention

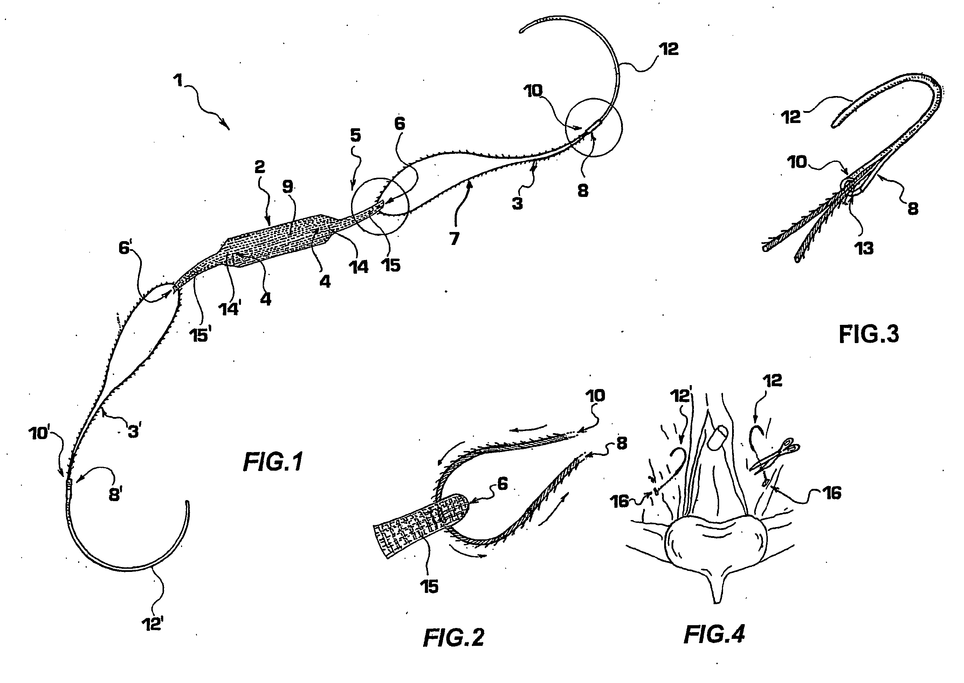

Referring initially to figure 1, a first embodiment of a prosthesis according to the invention is generally denoted by 1.

The prosthesis 1 comprises a main central body 2 realizing the support for the urethra; at the two opposite ends of the body 2 there are linked, in a manner that will be described hereinafter, two self-locking thread-shaped members 3, 3', by which said central body 2 is positioned to support the urethra.

The body 2 is made of a biocompatible material and is apt to be placed in a position substantially underlying at least the middle urethra.

In particular, in the embodiment considered herein, the main body 2 is designed for the treatment of first-, second- or third-degree stress urinary incontinence (SUI), depending on the specific extension of the body 2 itself. Just depending on such an extension, the body 2 could be placed even under the bladder body and base, beside under the middle urethra.

The central body 2 may be made of a non-reabsorbable (e.g. polypropylene) or partially reabsorbable (e.g., polypropylene and glycolide) material, or of a biologic material (e.g., pericardium).

In the embodiment depicted here, the central body 2 has a mesh-like structure 9 of non-reabsorbable polypropylene (C₃H₆)ₙ monofilaments, and having a substantially oblong shape. In correspondence of opposite longitudinal ends 4, 4' thereof, the central body 2 has two extensions 5, 5'. Each of said extensions 5, 5' comprises a first gradual narrowing, respectively denoted by number references 14, 14', ending with an appendage 15, 15', respectively, having substantially tapered contour.

Substantially in correspondence of a terminal end 6, 6' of each appendage 15, 15', a linking is made between a respective thread-shaped member 3, 3' and the body 2. By said linking, the thread-shaped members 3, 3' are attached to the body 2 itself and can therefore exert traction thereon, thereby allowing a correct positioning and tensioning of the prosthesis 1 and thus implementing means for positioning the latter.

The modes of connecting each thread-shaped member 3, 3' to the main body 2 are depicted in greater detail in figure 2.

Referring to the latter figure, at the making of the.prosthesis 1 a first terminal end 8 of a thread-shaped element, in the present example the member denoted by 3, is inserted through a weave of the mesh 9 of the respective appendage 15, and subsequently attached to a second terminal end 10 of the thread-shaped member 3 itself. Thus, the linking between body 2 and thread-shaped member 3 occurs substantially at the middle point of the latter. Likewise, the thread-shaped member 3' is linked substantially in correspondence of an end 6' of the respective appendage 15'. In this case as well, a first end 8' of the thread-shaped member 3', upon crossing the weave of the mesh 9, is attached to a second end 10'.

Therefore, in the present example the connection between each thread-shaped member 3, 3' and main body 2 is made so that the element 3, 3' itself describes a loop or a ring and is connected to the body 2 in correspondence of a race or arc of said loop or ring, so that each member 3, 3' defines a twin member for tensioning the main body 2.

It will be appreciated that the linking between thread-shaped members 3, 3' and central body 2 by interposition of the tapered extensions 5, 5' allows even distribution of the stresses generating during the traction of the body 2 itself. Linking the body 2 directly at its central portion would generate therein a state of three-axial tension that could cause the forming of folds, the onset of unevenly stressed zones or anyhow the kinking of the former.

Each thread-shaped member 3, 3' has, along its own axial development, a plurality of inclination 7, whose spike-like members is such as not to generate contrast with neighboring tissues and organs during the positioning of the prosthesis, and such as not to allow over time a relaxation thereof that would cause its loss of efficiency. In particular, at the two tensioning sections of each of the above-defined member 3, 3' said spike-like members 7 have substantially opposite inclinations, just to ensure a proper grip on the tissues.

As it is shown in greater detail in figure 3, the joined ends 8-10 and 8'-10' of each thread-shaped member 3, 3' may be assembled inside a means for insertion in the patient's body, and particularly in correspondence of a respective curved needle 12, 12', having for this purpose a mouth 13, 13', and be secured thereto by pressing of said mouth 13, 13'.

According to a variant embodiment, the two joined ends 8-10 and 8'-10' of the elements 3, 3' may be left free. In this latter case, the insertion into the patient's body and the dragging of the members 3,3' during the operation may be carried out by use of external instruments (like, e.g., needles).

The thread-shaped members 3, 3' may be made of a non-absorbable (e.g., polypropylene), partially absorbable (e.g., polypropylene and glycolide) or absorbable (e.g., polydioxanone) material.

According to a preferred execution, the operation of implantation of such a prosthesis 1, in this case designed for therapy of first- second- or third degree urinary incontinence, occurs by vaginoperineal approach with the catheterized patient in a lithotomic position pushed into Trendelenburg position. Anesthesia may be preferably local, local-regional or general.

Always according to a preferred execution, a median longitudinal incision of about 1 cm is made in the anterior wall of the vagina, at about 1 cm from the urethral tubercle. Vaginal mucosa, vaginal muscular mucosa and fascia are incised, a paraurethral beveled dissection is made up to the middle margin of the ischiopubic ramus (1 cm lateral to the urethra with a 45-degree direction with respect to the ischiopubic ramus). Needle 12 of the prosthesis 1 is mounted on a needle holder and passed through the tunnel prepared beforehand, and then through the pubocervical fascia, the musculus obturator internus, to the obturator fascia, transiting close to the posterior margin of the ischiopubic ramus, to make it emerge at the inguinal skin, through the obturator foramen at the inferomedial margin thereof. The point of emergence of the needle 12 corresponds to the intersection of two straight lines, one going through the base of the clitoris and one through the inguinal folds.

The operation is repeated on the other side. Traction is applied to the thread-shaped members 3, 3', and the central part of the prosthesis 1 is positioned under the middle urethra.

The self-locking thread-shaped members 3, 3' allow to adjust and support the prosthesis 1 until fibrosis is attained, to then reabsorb gradually, with the result of a remarkable reduction of implanted material. Continence is tested by stress test (e.g., coughs with 250 ml in bladder) with the main body 2 "loose" and then in position. A "Tension-Free" check is performed (about 3-4 mm between main body 2 and urethra). Vaginal breach is sutured with reabsorbable material. Then, the members 3, 3' are cut at skin emergence and the operation is concluded with suitable medications.

Figure 4 shows just the last phase of the surgery for *in situ* implantation of the prosthesis 1. In particular, there are highlighted two incisions 16, 16' from which the self-locking threads 3, 3' emerge. As mentioned above, subsequently to having tensioned said central body 2 (not visible) to support the middle urethra by applying traction to the threads themselves, they are cut at the skin emergence spot.

There will now be briefly described a second embodiment of the prosthesis of the invention, which can advantageously be used to solve with a single surgery the problems of urinary incontinence and of urogenital prolapse. This further embodiment will be described only referring to the elements differentiating it from the first embodiment already disclosed.

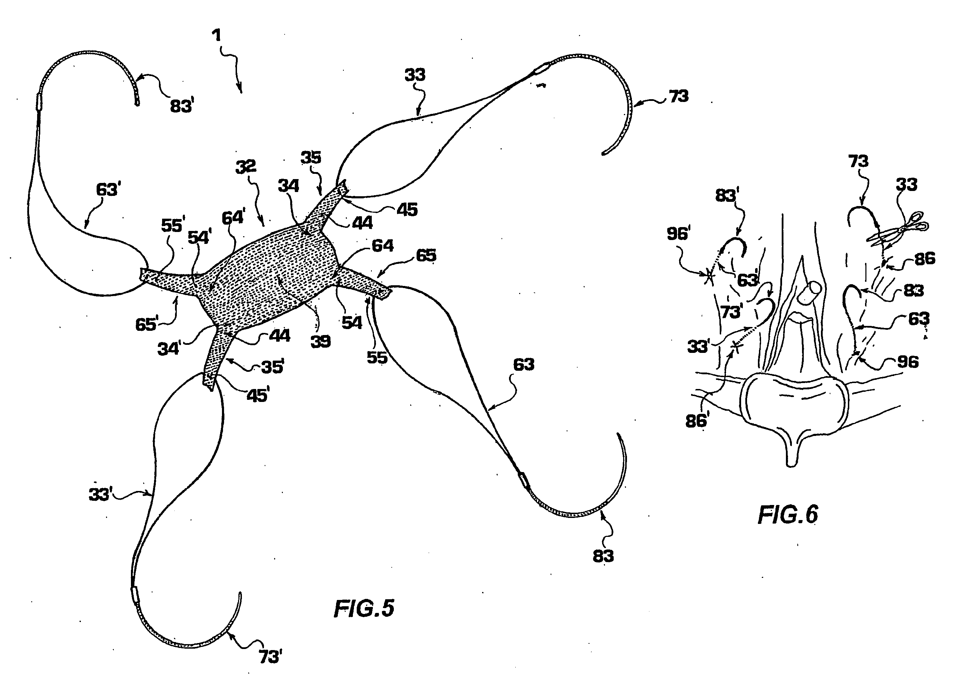

Referring to figures 5 and 6, a prosthesis 1' comprises a central body 32 to which there are linked, in the aforedescribed manner, four self-locking thread-shaped members 33, 33', 63 and 63', by which the central body 32 is positioned to support the urethra and the body and base of the bladder.

The main body 32 has a substantially quadrilateral shape, and said thread-shaped members 33, 33', 63 and 63' originate therefrom substantially in correspondence of the vertexes of a quadrilateral.

Otherwise, the main body 32 and the members 33, 33', 63 and 63' may have a realization in all analogous to that described hereto with reference to the first embodiment.

It will be appreciated that in the present case the presence of four thread-shaped members, rather than two, for making the positioning means serves to ensure a correct *in situ* positioning of the prosthesis and a correct tensioning and support thereof.

Analogously to what has been described hereto, the thread-shaped members 33, 33', 63, 63' comprise along their axial development a plurality of spike-like members (not depicted) similar to those disclosed hereto.

Always analogously to what has been described hereto, substantially in correspondence of its own opposite ends 34, 34', and 64, 64' defining the vertexes of a quadrilateral, the central body 32 has respective extensions 35, 35' and 65, 65' analogous to those already disclosed.

In figure 6 it is schematically depicted the last phase of the surgical operation for the positioning of said prosthesis 1'. In particular, there are highlighted four incisions 86, 86' and 96, 96' from which the self-locking members, respectively 33, 33' and 63, 63', emerge. Subsequently to having tensioned said central body 32 (not visible) by applying traction to the threads themselves, they are cut at the skin emergence spot.

The present invention has hereto been described with reference to two preferred embodiments thereof. It is understood that other embodiments might exist, all falling within the same inventive kernel, and all comprised within the protective scope of the claims hereinafter.

## Claims

1. An urogenital prosthesis (1) to be used in surgical therapy of female prolapse and/or urinary incontinence, comprising:
- a main body (2), made of a biocompatible material and apt to be placed in a position substantially underlying at least the middle urethra;
- positioning means, originating from said main body (2) and apt to be maneuvered to allow the positioning and the correct tensioning of the prosthesis (1) during the *in situ* implantation thereof,
wherein said positioning means comprises at least one pair of elements (3, 3') of substantially thread-shaped conformation, arranged at opposite sides of said main body (2),
wherein each of said thread-shaped members (3, 3') is connected to said main body (2) by crossing the weave of the latter,
wherein each of said thread-shaped members (3, 3') describes substantially a loop or a ring, it being connected to said main body in correspondence of a race of said loop or of an arc of ring, so as to define a double member for tensioning said main body (2), and
**characterized in that** said main body (2) comprises, in correspondence of each portion connecting with said thread-shaped members (3, 3'), a respective oblong extension (5, 5') of substantially tapered conformation, each of said extensions comprising a first gradual narrowing (14, 14') ending with an appendage (15, 15') having substantially tapered contour.

2. The prosthesis (1) according to the preceding claim, wherein said body (2) has an extension such as to allow a placement thereof under the bladder body and base, and under the middle urethra.

3. The prosthesis (1) according to claims 1 or 2, wherein said biocompatible material has a substantially mesh-like structure (9).

4. The prosthesis (1) according to the preceding claim, wherein said mesh-like structure is made by means of a synthetic polypropylene monofilament.

5. The prosthesis (1) according to any one of the preceding claims, wherein said main body (2) has a substantially oblong shape.

6. The prosthesis (1) according to any one of the preceding claims, wherein said main body (2) has a substantially oval shape.

7. The prosthesis (1) according to any one of the preceding claims, wherein said positioning means comprises four thread-shaped members (33, 33', 63, 63'), originating from said main body (32) substantially in correspondence of the vertexes of a quadrilateral.

8. The prosthesis (1) according to any one of the preceding claims, wherein said thread-shaped members (3, 3') are of self-locking type (3).

9. The prosthesis (1) according to the preceding claim, wherein said self-locking thread-shaped members (3, 3') have spike-like members.

10. The prosthesis (1) according to any one of the preceding claims, wherein said thread-shaped members (3, 3') are made of a reabsorbable or partially reabsorbable material.

11. The prosthesis (1) according to any one of the preceding claims, wherein each of said thread-shaped members (3, 3') is connected to said main body (2) by linking.

12. The prosthesis (1) according to any one of the preceding claims, which is preset with means (12) for insertion in a patient's body connected to said thread-shaped members (3, 3') in correspondence of respective ends of the latter arranged oppositely with respect to their connection to said main body (2).

13. The prosthesis (1) according to the preceding claim, wherein said insertion means (12) comprises at least one curved needle (12).

## Patentansprüche

1. Urogenitalprothese (1) zur Verwendung bei der chirurgischen Behandlung von weiblichem Prolaps und/oder Harninkontinenz, mit:
- einem Hauptkörper (2), der aus einem biokompatiblen Material besteht und in einer Position platzierbar ist, in welcher er wenigstens der mittleren Urethra im Wesentlichen unterlagert ist;
- einer Positioniereinrichtung, die von dem Hauptkörper (2) ausgeht und manövrierbar ist, um das Positionieren und das korrekte Spannen der Prothese (1) während der in situ Implantation derselben zu erlauben,
wobei die Positioniereinrichtung wenigstens ein Paar Elemente (3, 3') von im Wesentlichen fadenförmiger Gestaltung umfasst, die auf entgegengesetzten Seiten des Hauptkörpers (2) angeordnet sind,
wobei jedes der fadenförmigen Teile (3, 3') mit dem Hauptkörper (2) durch Kreuzen des Gewebes von letzterem verbunden ist,
wobei jedes der fadenförmigen Teile (3, 3') im Wesentlichen eine Schleife oder einen Ring beschreibt, die bzw. der mit dem Hauptkörper entsprechend einem Bogen der Schleife oder einem Bogen des Ringes verbunden ist, so dass ein doppeltes Teil zum Spannen des Hauptkörpers (2) gebildet ist, und
**dadurch gekennzeichnet, dass** der Hauptkörper (2) entsprechend jedem Teil, der mit den fadenförmigen Teilen (3, 3') verbunden ist, einen länglichen Fortsatz (5, 5') von im Wesentlichen konisch zulaufender Gestaltung aufweist, wobei die Fortsätze einen ersten allmählich schmaler werdenden Teil (14, 14') aufweisen, der in einem Ansatz (15, 15') endet, welcher eine im wesentlichen konisch zulaufende Kontur hat.

2. Prothese (1) nach dem vorhergehenden Anspruch, wobei der Körper (2) eine derartige Ausdehnung hat, dass er unter dem Blasenkörper und der Blasenbasis sowie unter der mittleren Urethra platziert werden kann.

3. Prothese (1) nach Anspruch 1 oder 2, wobei das biokompatible Material eine im Wesentlichen gitterartige Struktur (9) hat.

4. Prothese (1) nach dem vorhergehenden Anspruch, wobei die gitterartige Struktur mit Hilfe eines synthetischen Polypropylenmonofilaments hergestellt ist.

5. Prothese (1) nach einem der vorhergehenden Ansprüche, wobei der Hauptkörper (2) eine im Wesentlichen längliche Form hat.

6. Prothese (1) nach einem der vorhergehenden Ansprüche, wobei der Hauptkörper (2) eine im Wesentlichen ovale Form hat.

7. Prothese (1) nach einem der vorhergehenden Ansprüche, wobei die Positioniereinrichtung vier fadenförmige Teile (33, 33', 63, 63') aufweist, die von dem Hauptkörper (32) im Wesentlichen in Entsprechung zu den Ecken eines Vierecks ausgehen.

8. Prothese (1) nach einem der vorhergehenden Ansprüche, wobei die fadenförmigen Teile (3, 3') von selbstverriegelnder Bauart (3) sind.

9. Prothese (1) nach dem vorhergehenden Anspruch, wobei die selbstverriegelnden fadenförmigen Teile (3, 3') stachelartige Teile haben.

10. Prothese (1) nach einem der vorhergehenden Ansprüche, wobei die fadenförmigen Teile (3, 3') aus einem resorbierbaren oder einem teilweise resorbierbaren Material hergestellt sind.

11. Prothese (1) nach einem der vorhergehenden Ansprüche, wobei jedes der fadenförmigen Teile (3,3') mit dem Hauptkörper (2) durch Bindung verbunden ist.

12. Prothese (1) nach einem der vorhergehenden Ansprüche, welche vorausgerüstet ist mit einer Einrichtung (12) zur Einführung in den Körper eines Patienten, welche mit den fadenförmigen Teilen (3, 3') in Entsprechung der Enden von letzteren, die entgegengesetzt zu ihrer Verbindung mit dem Hauptkörper (2) angeordnet sind, verbunden ist.

13. Prothese (1) nach dem vorhergehenden Anspruch, wobei die Einführeinrichtung (12) wenigstens eine gekrümmte Nadel (12) umfasst.

## Revendications

1. Prothèse urogénitale (1) destinée à être utilisée dans le traitement chirurgical des prolapsus et/ou incontinence urinaire féminine, comprenant :
- un corps principal (2), constitué d'un matériau biocompatible et apte à être placé dans une position substantiellement sous-jacente au moins à l'urètre moyen ;
- un moyen de positionnement, commençant sur ledit corps principal (2) et apte à être manoeuvré pour permettre le positionnement et la mise en tension correcte de la prothèse (1) au cours de son implantation *in situ,*
dans laquelle ledit moyen de positionnement comprend au moins une paire d'éléments (3, 3') ayant substantiellement une structure de fil, placés sur des côtés opposés dudit corps principal (2),
dans laquelle chacun desdits éléments en forme de fil (3, 3') est connecté audit corps principal (2) en croisant l'armature de ce dernier,
dans laquelle chacun desdits éléments en forme de fil (3, 3') décrit substantiellement une boucle ou un anneau, connecté audit corps principal en correspondance avec une piste de ladite boucle ou avec un arc de l'anneau, afin de définir un élément double pour tendre ledit corps principal (2), et
**caractérisée en ce que** ledit corps principal (2) comprend, en correspondance avec chaque partie se connectant auxdits éléments en forme de fil (3, 3'), une extension oblongue respective (5, 5') de structure substantiellement conique, chacune desdites extensions comprenant un premier rétrécissement progressif (14, 14') se terminant par un appendice (15, 15') ayant un profil substantiellement conique.

2. Prothèse (1) selon la revendication 1, dans laquelle ledit corps (2) a une extension destinée à permettre une mise en place de celui-ci sous le corps et la base de la vessie, et sous l'urètre moyen.

3. Prothèse (1) selon la revendication 1 ou 2, dans laquelle ledit matériau biocompatible a substantiellement une structure de filet (9).

4. Prothèse (1) selon la revendication précédente, dans laquelle ladite structure de filet est constituée au moyen d'un monofilament en polypropylène synthétique.

5. Prothèse (1) selon l'une quelconque des revendications précédentes, dans laquelle ledit corps principal (2) a une forme substantiellement oblongue.

6. Prothèse (1) selon l'une quelconque des revendications précédentes, dans laquelle ledit corps principal (2) a une forme substantiellement ovale.

7. Prothèse (1) selon l'une quelconque des revendications précédentes, dans laquelle ledit moyen de positionnement comprend quatre éléments en forme de fil (33, 33', 63, 63'), commençant audit corps principal (2), substantiellement en correspondance avec les sommets d'un quadrilatère.

8. Prothèse (1) selon l'une quelconque des revendications précédentes, dans laquelle lesdits éléments en forme de fil (3, 3') sont du type autobloquant (3).

9. Prothèse (1) selon la revendication précédente, dans laquelle lesdits éléments en forme de fil autobloquants (3, 3') ont des éléments en forme d'épines.

10. Prothèse (1) selon l'une quelconque des revendications précédentes, dans laquelle lesdits éléments en forme de fil (3, 3') sont faits d'un matériau re-résorbable ou partiellement re-résorbable.

11. Prothèse (1) selon l'une quelconque des revendications précédentes, dans laquelle chacun desdits éléments en forme de fil (3, 3') est connecté audit corps principal (2) par une articulation.

12. Prothèse (1) selon l'une quelconque des revendications précédentes, munie d'un moyen (12) permettant l'insertion dans le corps d'un patient connecté auxdits éléments en forme de fil (3, 3') en correspondance avec les extrémités respectives de ces derniers placés de façon opposée par rapport à leur connexion au corps principal (2).

13. Prothèse (1) selon la revendication précédente, dans laquelle ledit moyen d'insertion (12) comprend au moins une aiguille incurvée (12).
